# EUROPEAN PATENT APPLICATION

(11) **EP 4 509 502 A1**
(43) Date of publication of application: **19.02.2025**
(21) Application number: 23787622.2
(22) Date of filing: 10.04.2023
(51) Int. Cl.: C07D 403/12, A61K 31/506, A61P 35/00

(54) **CRYSTAL FORM OF FGFR4 SELECTIVE INHIBITOR COMPOUND, AND PREPARATION METHOD THEREFOR AND USE THEREOF**

(30) Priority: 14.04.2022 CN 202210393796
(71) Applicant: Zhe Jiang Hisun Pharmaceutical Co., Ltd., Taizhou, Zhejiang 318000 (CN)
(72) Inventor: ZHENG, Jianfeng, Taizhou, Zhejiang 318000 (CN); SHI, Zhenjuan, Taizhou, Zhejiang 318000 (CN); SU, Di, Taizhou, Zhejiang 318000 (CN); GONG, Yongxiang, Taizhou, Zhejiang 318000 (CN); ZHONG, Jinqing, Taizhou, Zhejiang 318000 (CN)
(74) Representative: HGF
(86) International application number: PCT/CN2023/087201
(87) International publication number: WO 2023/197978

(57) **Abstract**

The present invention relates to a crystal form A of a compound of formula (I), and a preparation method therefor and the use thereof. The crystal form A has good properties in terms of physical and chemical stability, water solubility, bioavailability and processing adaptability.

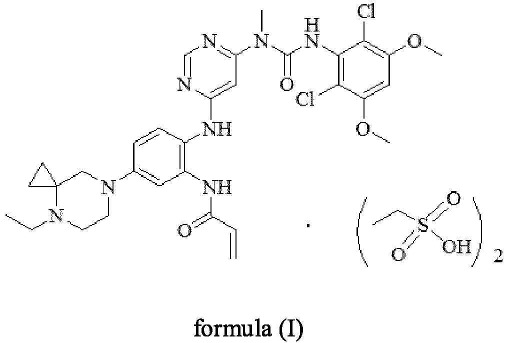

## Description

This application claims the priority of Chinese Patent Application No. 202210393796.6, filed with the China National Intellectual Property Administration on April 14, 2022, and titled with "CRYSTAL FORM OF FGFR4 SELECTIVE INHIBITOR COMPOUND, AND PREPARATION METHOD THEREFOR AND USE THEREOF", the disclosure of which is hereby incorporated by reference in its entirety.

### FIELD

The present disclosure relates to the technical field of pharmaceutical chemistry, and in particularly relates to a new crystal form of N-(2-((6-(3-(2,6-dichloro-3,5-dimethoxyphenyl)-1-methylureido)pyrimidin-4-yl)amino)-5-(4-eth yl-4,7-diazaspiro[2.5]octan-7-yl)phenyl)acrylamide diethanesulfonate salt, a preparation method therefor, and medical use thereof.

### BACKGROUND

PCT/CN2017/085135 reports a new type of pyrimidine derivatives, which are inhibitors against the FGFR4 pathway. A large of evidence suggests that gene amplification mutations in FGFR4 are present in lung cancer, ovarian cancer, prostate cancer, liver cancer and cholangiocarcinoma, and so on. Compared to other FGFR inhibitors, selective FGFR4 inhibitors have the advantage of low toxicity (Brow, AP et al (2005), Toxcol. Pathol., 449-455). Considering the deficiency of currently available drugs in terms of safety and efficacy, and especially considering the application potential of selective FGFR4 inhibitors, the current research on selective FGFR4 inhibitors in anti-cancer, especially anti-liver cancer and other aspects is far from enough, it is therefore desirable to investigate and develop novel FGFR4 inhibitors.

PCT/CN2017/085135 discloses a free-base compound as represented by formula (II) with the chemical name of N-(2-((6-(3-(2,6-dichloro-3,5-dimethoxyphenyl)-1-methylureido) pyrimidin-4-yl)amino)-5-(4-ethyl-4,7-diazaspiro[2.5]octane-7-yl)phenyl)acrylamide, having the structure shown below:

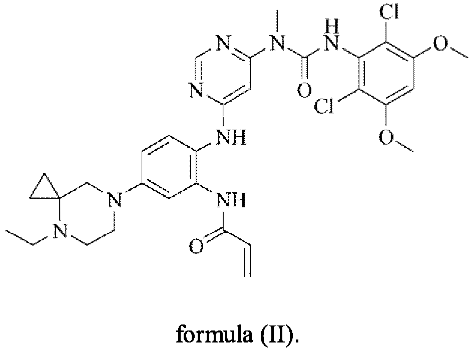

In vitro cell activity assay showed that a compound as represented by formula (II) has good inhibitory activity against hepatocellular carcinoma cells Hep3B with an IC50 value of 0.019 µM, having a good prospect for development. However, in the course of investigating the drugability of the compound as represented by formula (II), the inventor of the present disclosure found that the common pharmaceutically acceptable salts of the compound as represented by formula (II) are unsatisfactory in terms of physicochemical properties and drugability, including bioavailability, water-solubility, hygroscopicity, and the like. Therefore, it is necessary to conduct in-depth investigation to find new forms thereof, being suitable for medicinal purposes, to meet the needs of drug development.

### SUMMARY

Considering the drawbacks of the prior art, one of the objectives of the present disclosure is to provide a new form of the compound, to overcome at least one of the above mentioned drawbacks of the prior art. To this end, the present disclosure provides a crystal form A of a compound represented by formula (I), with good chemical and physical stability:

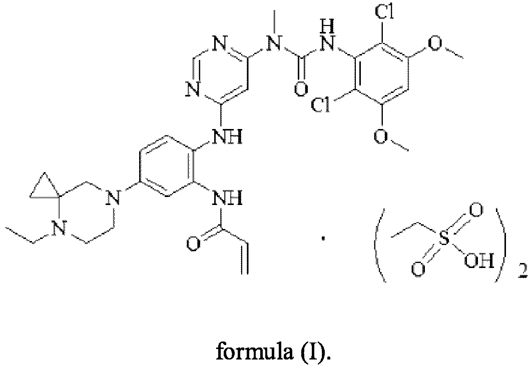

The crystal form has excellent physical and chemical stability, water-solubility, bioavailability and hygroscopicity, the preparation method of the crystal form of the present disclosure is simple and easy to operate, and is easy to realize industrial production.

The X-ray powder diffraction (XRPD) pattern of the crystal form A of the compound represented by formula (I) in the present disclosure comprises characteristic peaks at diffraction angle 2θ of 6.6±0.2°, 14.6±0.2°, 16.4±0.2°, 17.6±0.2°, 19.6±0.2°, 20.3±0.2°, 20.8±0.2°, 23.0±0.2°, and 26.9±0.2°. Preferably, the crystal form A of the compound represented by formula (I) of the present disclosure further comprises characteristic peaks at diffraction angle 2θ of 22.1±0.2°, 23.3±0.2°, 25.8±0.2°, 28.2±0.2°, and 29.8±0.2°.

More preferably, the X-ray powder diffraction (XRPD) pattern of the crystal form A of the compound represented by formula (I) in the present disclosure further comprises characteristic peaks at 2θ of 7.2±0.2°, 10.2±0.2°, 13.4±0.2°, 19.1±0.2°, 24.6±0.2°, 25.0±0.2° and 30.9±0.2°.

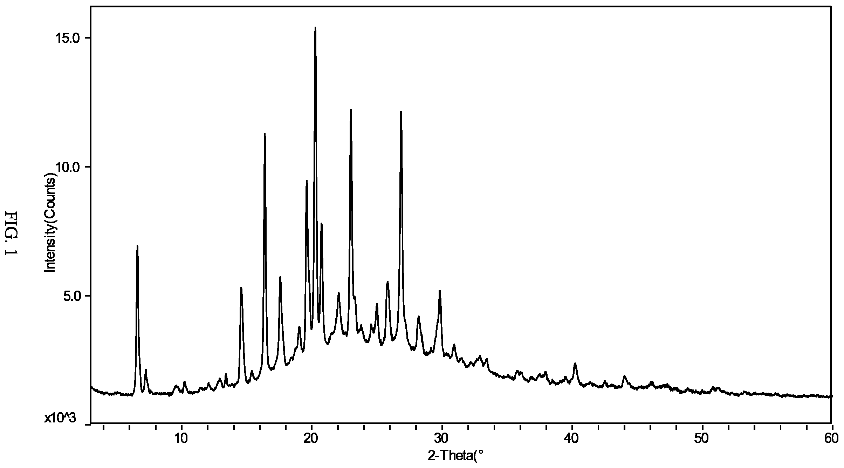

Preferably, the crystal form A of the compound represented by formula (I) of the present disclosure has substantially the same X-ray powder diffraction pattern as shown in FIG. 1.

In one embodiment, the data of 2θ and relative intensity for X-ray powder diffraction pattern is shown in Table 1 below.

**Table 1**

| **number of peak** | **2θ (°)** | **relative intensity (%)** |
|---|---|---|
| 1 | 6.6±0.2° | 46.4 |
| 2 | 7.2±0.2° | 7.6 |
| 3 | 10.2±0.2° | 3.5 |
| 4 | 13.4±0.2° | 4.2 |
| 5 | 14.6±0.2° | 30.2 |
| 6 | 16.4±0.2° | 75 |
| 7 | 17.6±0.2° | 28 |
| 8 | 19.1±0.2° | 8.3 |
| 9 | 19.6±0.2° | 48 |
| 10 | 20.3±0.2° | 100 |
| 11 | 20.8±0.2° | 35.5 |
| 12 | 22.1±0.2° | 14.1 |
| 13 | 23.0±0.2° | 70.5 |
| 14 | 23.3±0.2° | 12.4 |
| 15 | 24.6±0.2° | 5.5 |
| 16 | 25.0±0.2° | 12 |
| 17 | 25.8±0.2° | 18.3 |
| 18 | 26.9±0.2° | 72.3 |
| 19 | 28.2±0.2° | 10.8 |
| 20 | 29.8±0.2° | 20.5 |
| 21 | 30.9±0.2° | 4.7 |

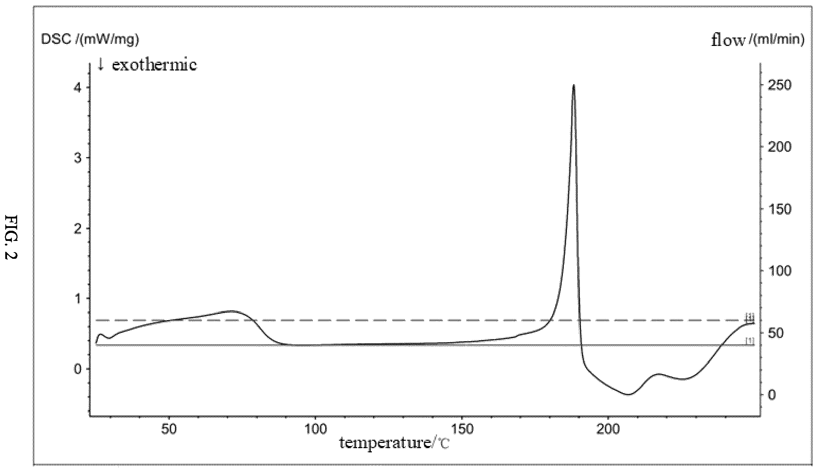

Preferably, the crystal form A of the compound represented by formula (I) of the present disclosure has substantially the same DSC trace as shown in FIG. 2, and it has a melting point of 188°C.

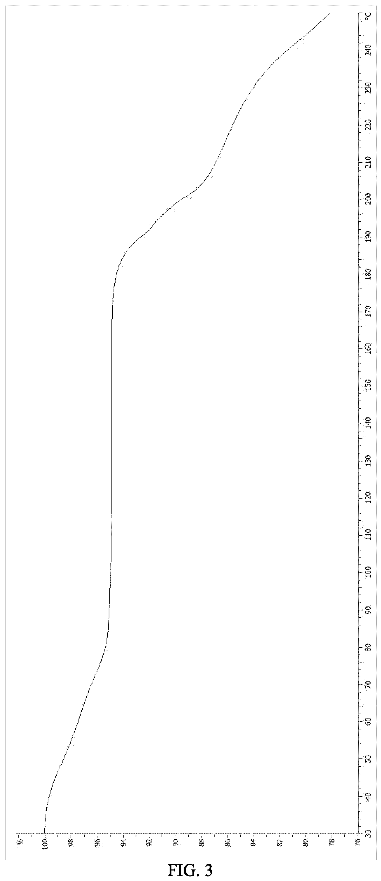

Preferably, the crystal form A of the compound represented by formula (I) of the present disclosure has substantially the same TGA profile as shown in FIG. 3.

Another objective of the present disclosure is to provide a method for preparing the crystal form A of the compound represented by formula (I), and the method comprises the following steps:

providing a suspension of the compound represented by formula (I), stirring at 10°C to 40°C for 4 h to 12 h, and filtering to obtain the crystal form A of the compound represented by formula (I).

Preferably, the suspension of the compound represented by formula (I) is a suspension of a compound represented by formula (I) in a solvent selected from the group consisting of an alcohol, a ketone, an ester, ethers, an alkane, water, and a mixture thereof.

Preferably, the suspension has a solid-liquid ratio (g/mL) of 1:3-40.

Preferably, the method further comprises a step of drying after filtrating.

The present disclosure further relates to a pharmaceutical composition comprising the crystal form A of the compound represented by formula (I), wherein the pharmaceutical composition comprises a therapeutically effective amount of the crystal form A of the compound represented by formula (I) and one or more pharmaceutically acceptable carriers.

The present disclosure further relates to use of the crystal form A of a compound represented by formula (I), or the pharmaceutical composition comprising the crystal form A of the compound represented by formula (I), in the manufacture of a medicament for the treatment a tumor. The tumor is preferably selected from the group consisting of non-small cell lung cancer, gastric cancer, multiple myeloma, liver cancer, and cholangiocarcinoma, more preferably liver cancer and cholangiocarcinoma.

Accordingly, the present disclosure further relates to a method for treating a tumor, which comprises administering the crystal form A of the compound represented by formula (I) of the present disclosure or a pharmaceutical composition comprising the crystal form A of the compound represented by formula (I) to a subject in need thereof. The tumor is preferably selected from the group consisting of non-small cell lung cancer, gastric cancer, multiple myeloma, liver cancer, and cholangiocarcinoma, more preferably liver cancer and cholangiocarcinoma.

The inventors of the present disclosure have discovered the crystal form A of the compound represented by formula (I) after a lot of research, which requires a simple crystallization process, is easy to operate, has low pollution, has a high yield, and can be industrially produced. Moreover, the crystal form drug of the present disclosure has the advantages of high product purity, excellent physical and chemical properties, good chemical stability, good water-solubility, high bioavailability, and low hygroscopicity.

### BRIEF DESCRIPTION OF DRAWINGS

FIG. 1 is the X-ray powder diffraction pattern of the crystal form A of the compound represented by formula (I) obtained in Example 1.
FIG. 2 is the DSC trace of the crystal form A of the compound represented by formula (I) obtained in Example 1.
FIG. 3 is the TGA profile of the crystal form A of the compound represented by formula (I) obtained in Example 1.

### DETAILED DESCRIPTION

The following examples are used to further illustrate the present disclosure, which do not constitute a restriction or limitation to the scope of the present disclosure.

The crude product of the compound represented by formula (I) in the embodiments of the present disclosure was obtained by homemade production.

The solvents used in the present disclosure are not particularly limited and can be commercially available conventional solvents, for example, the ethanol can be commercially available ethanol, including industrial ethanol, anhydrous ethanol and the like.

Unless otherwise specified, 'stirring' in the method of the present disclosure can be performed by conventional methods in the field, for example, stirring method includes magnetic stirring, and mechanical stirring, and stirring is performed at a speed of 50-300 rpm/min, preferably 100-200 rpm/min.

The X-ray powder diffractometer model used in the present disclosure was Rigaku D/max-2200 with Cu target, and the test conditions and the operation protocol included scanning speed of 4°/min, and scanning step size of 0.01°.

The differential scanning calorimeter model used in the present disclosure was NETZSCH DSC200 F3 Jaia; and test conditions included the heating rate of 10 °C/min and the temperature ranging from 25-250 °C.

The thermogravimetric analyzer (TGA) model used in the present disclosure was PerkinElmer TGA400, and the test conditions included the heating rate of 10°C/min and the temperature ranging from 30-250 °C.

The compound represented by formula (I) of the present disclosure was subjected to HPLC for purity detection under the following conditions. The used chromatographic column was Waters, XBridge C18 4.6×150 mm, 3.5 µm; the mobile phase A consisted of buffer solution: acetonitrile=70:30, the mobile phase B consisted of buffer solution: acetonitrile=35:35, the buffer solution was prepared by weighing 9.13 g of dipotassium hydrogen phosphate trihydrate in 2,000 mL of pure water, and adjusting pH using phosphoric acid to 8.0; the detection wavelength was 234 nm; the flow rate was 1.0 mL/min; the column temperature was 25°C; and the injection volume was 10µL.

### gradient elution conditions

| time (min) | A (%) | B (%) |
|---|---|---|
| 0 | 100 | 0 |
| 15 | 70 | 30 |
| 40 | 40 | 60 |
| 50 | 0 | 100 |
| 60 | 0 | 100 |
| 62 | 100 | 0 |
| 70 | 100 | 0 |

It should be emphasized that for the values or the numerical endpoints involved in the technical solutions of the present disclosure, the meaning or scope of protection is not limited to the numbers themselves, and those skilled in the art can understand that they include those that have been widely accepted permissible error ranges in the art, such as experimental errors, measurement errors, statistical errors and random errors, which are all included within the scope of the present disclosure.

### Example of preparing raw material

The crude product of the compound represented by formula (I) can be used as the raw material for the preparation of crystal form A in the method of the present disclosure. The raw material can be obtained by reacting the compound represented by formula (II) with ethanesulfonic acid. Specifically, the free-base compound represented by formula (II) (1000mg, 1.5mmol) was put into a reaction bottle, and 25ml of acetone and 1.25ml of water were added, stirred well, then ethanesulfonic acid (0.39g, 3.3mmol) was added, stirred until dissolution at room temperature, and the reaction solution was concentrated to remove acetone, filtered, and dried to obtain 1.2 g amorphous solid of the compound represented by formula (I), with a yield of 90%.

### Example 1 Preparation of the crystal form A of the compound represented by formula (I)

1.0 g of the crude product of the compound represented by formula (I) was added to 10 mL of methanol to form a suspension, warmed up to 30°C, stirred for 12 h, filtered, and dried in vacuum at 40°C to obtain 0.95g of crystal, with a purity of 99.64% detected by HPLC.

The X-ray powder diffraction pattern of this crystal is shown in FIG. 1, the DSC trace is shown in FIG. 2, and the TGA profile is shown in FIG. 3. This crystal was named as crystal form A of the compound represented by formula (I) in the present disclosure.

### Example 2 Preparation of the crystal form A of the compound represented by formula (I)

1.0 g of the crude product of the compound represented by formula (I) was added to 3 mL of water to form a suspension, stirred at 10°C for 4 h, filtered and dried in vacuum at 40°C to obtain 0.65 g of crystal, with a purity of 99.60% detected by HPLC. It was confirmed to be the crystal form A of the compound represented by formula (I) by X-ray powder diffraction (XRPD) pattern assay.

### Example 3 Preparation of the crystal form A of the compound represented by formula (I)

1.0 g of the crude product of compound represented by formula (I) was added to 10 mL of acetone to form a suspension, stirred at 20°C for 8 h, filtered and dried in vacuum at 40°C to obtain 0.94 g of crystal, with a purity of 99.53% detected by HPLC. It was confirmed to be the crystal form A of the compound represented by formula (I) by X-ray powder diffraction (XRPD) pattern assay.

### Example 4 Preparation of the crystal form A of the compound represented by formula (I)

1.0 g of the crude product of compound represented by formula (I) was added to 30 mL of ethyl acetate to form a suspension, stirred at 25°C for 6 h, filtered and dried in vacuum at 40°C to obtain 0.93 g of crystal, with a purity of 99.57% detected by HPLC. It was confirmed to be the crystal form A of the compound represented by formula (I) by X-ray powder diffraction (XRPD) pattern assay.

### Example 5 Preparation of the crystal form A of the compound represented by formula (I)

1.0 g of the crude product of compound represented by formula (I) was added to 20 mL of isopropyl ether to form a suspension, stirred at 15°C for 8 h, filtered and dried in vacuum at 40°C to obtain 0.96 g of crystal, with a purity of 99.47% detected by HPLC. It was confirmed to be the crystal form A of the compound represented by formula (I) by X-ray powder diffraction (XRPD) pattern assay.

### Example 6 Preparation of the crystal form A of the compound represented by formula (I)

1.0 g of the crude product of compound represented by formula (I) was added to 40 mL of n-heptane to form a suspension, stirred at 40°C for 4 h, filtered and dried in vacuum at 40°C to obtain 0.95 g of crystal, with a purity of 99.48% detected by HPLC. It was confirmed to be the crystal form A of the compound represented by formula (I) by X-ray powder diffraction (XRPD) pattern assay.

The crystal form A obtained in Examples 2-6 was tested to have the same XRD, DSC and TGA results as the crystal form A obtained in Example 1.

### Performance Example 1

Comparative solubility assay in water: the amorphous form and crystal form A of the compound represented by formula (I), and the compound represented by formula II (free base) were respectively subjected to solubility assay in water at 25°C, and the specific results are as follows:

| **sample** | **solubility in water at 25°C** |
|---|---|
| the amorphous form of the compound represented by formula (I) | 115mg/mL |
| the crystal form A of the compound represented by formula (I) | 100mg/mL |
| the compound represented by formula (II) (free base) | insoluble |

As can be seen from the above results, the solubility in water of the compound represented by formula (I) was much better than that of the free base compound represented by formula (II), indicating that the water-solubility was greatly improved after salt formation, the expected effect of salt formation was achieved, and the solubility in water of the amorphous salt and the crystal salt was relatively close to each other.

### Performance Example 2

Stability test: the amorphous form and the crystal form A of the compound represented by formula (I) were each placed under the conditions of 25°C, 40°C and light irradiation of 4500LX for 30 days, and the results were as follows.

| condition | stability at 25°C | | | | | | | | |
|---|---|---|---|---|---|---|---|---|---|
| | day 0 | | | day 10 | | | day 30 | | |
| detection item | crystal form | purity (%) | appearan ce | crystal form | purity (%) | appeara nce | crystal form | purity (%) | appeara nce |
| amorpho us form | amorph ous form | 98.80 | pale yellow powder | amorpho us form | 96.60 | yellow powder, partly caked | some characteris tic peaks of the crystal form A were showed | 91.60 | yellowis h brown powder, mostly caked |
| crystal form A | crystal form A | 99.64 | off-white powder | crystal form A | 99.63 | off-whit e powder | crystal form A | 99.62 | off-whit e powder |

| condition | stability at 40°C | | | | | | | | |
|---|---|---|---|---|---|---|---|---|---|
| | day 0 | | | day 10 | | | day 30 | | |
| detection item | crystal form | purity (%) | appearan ce | crystal form | purity (%) | appearanc e | crystal form | purity (%) | appear ance |
| amorpho us form | amorph ous form | 98.80 | pale yellow powder | some characteris tic peaks of the crystal form A were showed | 93.40 | yellowish brown powder, mostly caked | crystal form A | 87.60 | brown massiv e solid |
| crystal form A | crystal form A | 99.64 | off-white powder | crystal form A | 99.61 | off-white powder | crystal form A | 99.60 | off-wh ite powde r |

| condition | stability under the light irradiation of 4500LX | | | | | | | | |
|---|---|---|---|---|---|---|---|---|---|
| | day 0 | | | day 10 | | | day 30 | | |
| detection item | crystal form | purity (%) | appearan ce | crystal form | purity (%) | appeara nce | crystal form | purity (%) | appeara nce |
| amorpho us form | amorpho us form | 98.80 | pale yellow powder | amorpho us form | 86.20 | brown colloida 1 solid | amorpho us form | 73.70 | black viscous solid |
| crystal form A | crystal form A | 99.64 | off-whit e powder | crystal form A | 99.61 | off-whit e powder | crystal form A | 99.54 | off-whit e powder |

As can be seen from the above results of stability test, the amorphous form was prone to slow degradation under room temperature, rapid degradation under high temperature and light irradiation, especially under prolonged light irradiation, its appearance and purity have basically been destroyed. However, the tested crystal form was well preserved under conditions of room temperature, high temperature and light irradiation, and the difference between the two was obvious.

### Performance Example 3

Grinding test: The amorphous form and crystal form A were ground for 5 min each, and additionally ground under separate addition of ethanol or water to dryness. They were then subjected to XRPD, and the results are as follows:

| crystal form | crystal form A | | | amorphous form | | |
|---|---|---|---|---|---|---|
| grind condition | single grinding | grinding under the addition of ethanol | grinding under the addition of water | single grinding | grinding under the addition of ethanol | grinding under the addition of water |
| result | no change in crystal form | no change in crystal form | no change in crystal form | showing crystal transformation | showing crystal transformation | showing crystal transformation |

As can be seen from the grinding results, the amorphous from showed crystal transformation after grinding, indicating that it is susceptible to the risk of crystal transformation during preparation crushing or during granulation.

### Performance Example 4

### Pharmacokinetic experiment

On the day of the experiment, ICR mice in Group A were administered with 75 mg·kg -1 of a drug preparation of amorphous form of ethylsulfonate by single gavage, and ICR mice in Group B were administered with 75 mg·kg -1 of a drug preparation of crystal form A of ethylsulfonate by single gavage. Before and 0.25, 0.5, 1, 2, 4, 8, 10, 12 and 24 hours after administration, 0.15 mL blood was collected from fundus venous plexus and placed into anticoagulant tubes containing EDTA-K2.

The corresponding pharmacokinetic parameters were calculated using the non-atrioventricular model of Pharsight Phoenix 7.0, and the results are shown in the table below.

| pharmacokinetic parameters | unit | amorphous form | crystal form A |
|---|---|---|---|
| T_{1/2} | h | 1.37 | 1.52 |
| Tₘₐₓ | h | 0.500 | 0.610 |
| Cₘₐₓ | ng·mL⁻¹ | 4110 | 3985 |
| AUC₀₋ₜ | ng·h·mL⁻¹ | 9660 | 9342 |

It is well known in the art that the amorphous form typically has a higher energy due to the fact that it is in an unstable form and typically has significantly better bioavailability than the crystal form. However, as can be seen from the above results, the crystal form A has similar pharmacokinetic parameters to the amorphous form and exhibits essentially the same bioequivalence, which is unexpected.

## Claims

1. A crystal form A of a compound represented by formula (I), wherein an X-ray powder diffraction pattern of the crystal form A comprises characteristic peaks at diffraction angle 2θ of 6.6±0.2°, 14.6±0.2°, 16.4±0.2°, 17.6±0.2°, 19.6±0.2°, 20.3±0.2°, 20.8±0.2°, 23.0±0.2°, and 26.9±0.2°,

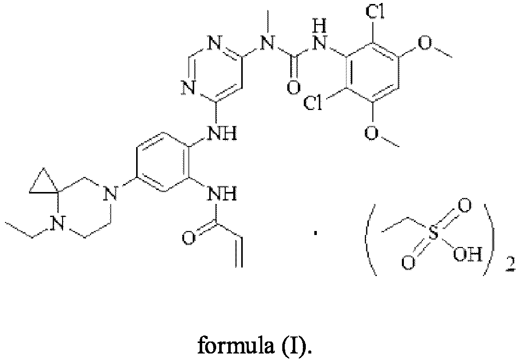

2. The crystal form A of the compound represented by formula (I) according to claim 1, wherein the X-ray powder diffraction pattern of the crystal form A further comprises characteristic peaks at diffraction angle 2θ of 22.1±0.2°, 23.3±0.2°, 25.8±0.2°, 28.2±0.2°, and 29.8±0.2°.

3. The crystal form A of the compound represented by formula (I) according to claim 2, wherein the X-ray powder diffraction pattern of the crystal form A further comprises characteristic peaks at diffraction angle 2θ of 7.2±0.2°, 10.2±0.2°, 13.4±0.2°, 19.1±0.2°, 24.6±0.2°, 25.0±0.2°, and 30.9±0.2°.

4. The crystal form A of the compound represented by formula (I) according to any one of claims 1 to 3, wherein the crystal form A has an X-ray powder diffraction pattern as shown in FIG. 1.

5. The crystal form A of the compound represented by formula (I) according to any one of claims 1 to 4, wherein the crystal form A has a melting point of 188°C, preferably, the crystal form A has a DSC trace as shown in FIG. 2.

6. The crystal form A of the compound represented by formula (I) according to any one of claims 1 to 5, wherein the crystal form A has a TGA profile as shown in FIG. 3.

7. A pharmaceutical composition comprising the crystal form A of the compound represented by formula (I) according to any one of claims 1 to 6.

8. Use of the crystal form A of the compound represented by formula (I) according to any one of claims 1 to 6 or the pharmaceutical composition according to claim 7 in the manufacture of a medicament for the treatment a cancer.
